# EUROPEAN PATENT APPLICATION

(11) **EP 3 906 878 A1**
(43) Date of publication of application: **10.11.2021**
(21) Application number: 21172084.2
(22) Date of filing: 04.05.2021
(51) Int. Cl.: A61B 18/14

(54) **ELECTRO-SURGICAL DEVICE WITH STABLE JAW TEMPERATURE**

(30) Priority: 07.05.2020 US 202063021394 P
(71) Applicant: Gyrus ACMI, Inc. d/b/a Olympus Surgical Technologies America, Westborough, MA 01581 (US)
(72) Inventor: Batchelor, Kester Julian, Mound, MN 55364 (US); Wang, Huisun, Maple Grove, MN 55311 (US)
(74) Representative: Noack, Andreas

(57) **Abstract**

Systems and techniques for a medical apparatus, specifically a handheld electrosurgical device, are described herein. In an example, the device includes an electrosurgical end effector, a temperature sensor to measure a temperature of the end effector, a cooler to cool the end effector based on the measured temperature, and a heater to heat the end effector based on the measured temperature. The end effector may be kept within a temperature range or band such as by allowing the end effector to be heated or cooled as desired during a surgical procedure.

## Description

### CROSS-REFERENCE TO RELATED APPLICATION

This application claims the benefit of priority to U.S. Provisional Patent Application Serial No. 63/021,394, filed May 7, 2020, the contents of which are incorporated herein in their entirety.

### TECHNICAL FIELD

The present disclosure relates to electrosurgical medical devices.

### BACKGROUND

Electrosurgery involves various techniques that can be used during medical procedures, such as, for example, laparoscopic surgery. The techniques may include, cutting, clamping, coagulating, desiccating, fulgurating, or the like, of biological tissue. These techniques may be used in procedures such as appendectomies, cholecystectomies, colectomies, cystectomies, gastric banding, gastric bypass, hernia repair, nephrectomy, Nissen fundoplication, prostatectomies, sleeve gastrectomy, or other similar procedures.

During electrosurgery, signals can be generated by an electrosurgical generator and provided to the biological tissue through an electrosurgical device. The electrosurgical energy can be provided to tissue via an end effector of the electrosurgical device. The end effector may include, for example, forceps, a conductive spatula, a j-hook, electrical pads, a needle, a blade, or the like. Different medical procedures can use different electrotherapeutic signals so as to achieve results specific to these different medical procedures. Various electrical metrics of the electrotherapeutic signals provided to the biological tissue being treated can be used to characterize these electrotherapeutic signals. These electrical metrics can include: polarity (e.g., monopolar, bipolar), AC and/or DC, frequency, signal amplitude, attack and decay profiles, or the like. Depending on the medical procedure and the end effector being used, the temperature at the end effector may be affected.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIGS. 1A and 1B illustrate an example of a system for a handheld electrosurgical device.
FIG. 2 illustrates a flowchart showing an example of a technique for determining the size of a blood vessel by stabilizing the jaw temperature of a handheld electrosurgical apparatus and detecting an initial impedance.
FIG. 3 illustrates an example of a method for using an electrosurgical apparatus.

### DETAILED DESCRIPTION

This document describes, among other things, a handheld electrosurgical device with active heating and cooling based upon a sensed temperature.

Electrosurgery can involve manipulating biological tissue using an end effector of an electrosurgical device. For example, an end effector may include one or more of: a jaw, a forceps, a conductive spatula, a j-hook, electrical pads, or the like. The end effector can be located at or near the distal tip of the electrosurgical device such as at the distal end of an elongated shaft extending outward from a handpiece. Manipulating tissue can involve applying an electrotherapy signal such as to produce a desired change in biological tissue of a surgical patient. Energy at the end effector can be used to modify the biological tissue through, for example, localized heating, desiccating the tissue, changing the tissue structure, or destroying tissue at the cellular level. Modifying tissue can be accomplished, for example, by electrical energy strikes emitted by the electrosurgical device at the end effector, by heating the end effector, or the like, alone or with mechanical tissue manipulation such as grasping, cutting, or the like. Similarly, a medical procedure in which an electrosurgical device is used may have one or more electrotherapy phases, such as an interrogation phase, a heating phase, a drying phase, a cauterizing phase, or the like.

An electrosurgical electrical signal can be generated by an electrosurgical generator. An electrosurgical generator can produce a variety of electrosurgical waveforms, which, in turn, can be applied to obtain corresponding tissue effects, such as those described above. The electrosurgical generator may be connected to a handpiece such that the electrical signal can then be passed from the handpiece to the end effector, such as via conductors extending along the shaft between the handpiece and the end effector.

The electrosurgical signal from the generator to the end effector may cause the temperature at the end effector to change (e.g., to increase) while a medical procedure is performed. For example, during a process of grasping, grabbing, or gripping a piece of tissue between the jaws of an electrosurgical forceps and applying electrosurgical energy such as a current, the tissue may be heated, modified, and released. Upon releasing the tissue, the temperature at the end effector may have increased from an initial temperature, before grasping the tissue, to a new temperature after the grasping and applying electrosurgical energy. Such heating of the end effector may be caused by heat transfer from the previously-heated tissue to the end effector of the surgical device (e.g., to the forceps), and may raise the temperature of the end effector. This change in temperature at the end effector may be as much as 60 degrees Celsius, for example.

When the temperature at the end effector increases, numerous issues may arise. For example, it may be more difficult for a surgeon to get sense data back from the end effector. Also, the heat may thermally affect additional tissue other than the target tissue. Therefore, the surgeon may desire the end effector to cool (e.g., to return the end effector to its initial temperature or to an end effector temperature that is below a threshold temperature that may affect tissue) before proceeding with the procedure. This may be done through cycling the heating source or waiting a period of time for the end effector to cool on its own. Such passive cooling of the end effector may prolong the surgical procedure or expose undesired tissue to the uncooled end effector.

Likewise, only providing a way to cool the end effector without the ability to also provide heat to the end effector may also prolong the surgical procedure if the surgeon is left waiting for the temperature at the end effector to increase to a desired temperature in order to begin or resume the procedure.

The present inventors have recognized, among other things, that maintaining the temperature of the end effector within a desired range, temperature band, or the like can help solve such issues. For example, it may permit a surgeon to immediately grasp additional tissue without exposing the additional tissue to unwanted thermal effects from the end effector. Also, it may permit the surgeon to collect more accurate or reliable data from a sensor at the end effector. Automatically keeping the temperature of the end effector within a desired range can help allow a surgeon to control temperature at the end effector as needed without introducing a large temperature swing, change, or the like.

For example, an electrosurgical apparatus may include an electrosurgical end effector, a temperature sensor to measure a temperature of the end effector, a cooler to cool the end effector based on the measured temperature, and a heater to heat the end effector based on the measured temperature. The temperature sensor, the cooler and the heater can be configured to be coupled to a controller circuit. The controller circuit can be configured to use the heater to heat the end effector such as when the measured temperature is below a first temperature limit and to use the cooler to cool the end effector such as when the measured temperature is above a second temperature limit. The controller circuit may be included within an electrosurgical generator coupled to the electrosurgical apparatus, or it may be included within the electrosurgical apparatus itself.

The end effector may include, for example, a jaw or forceps, such as can include or be coupled to the temperature sensor, the cooler, or the heater. An irrigation conduit can be included, such as can be provided separately from the cooler. This may include an irrigation line, such as from an external fluid reservoir, which can be coupled to a conduit that can extend through the electrosurgical apparatus to the end effector to deliver a fluid (e.g., saline or the like) to the end effector.

For example, the cooler may include at least one of a cryogenic cooler, an air or other gas flow cooler, a fluid flow cooler, a thermoelectric cooler, an active suction cooler, a fluid flushing cooler, a closed-circuit fluid flow cooler, a treatment site irrigation conduit, among others. The heater may include at least one of a resistive heater, an optoacoustic heater, an electrosurgical electrode, a heat pump, a heat pipe, among others. The handheld electrosurgical apparatus may also include or be coupled to a user-feedback indicator, such as a light emitting diode (LED) other indicator light, a haptic feedback device, or the like, such as which can notify a user of whether the measured temperature is within a specified range. The user-feedback indicator may also be included, on the electrosurgical generator or a user interface device.

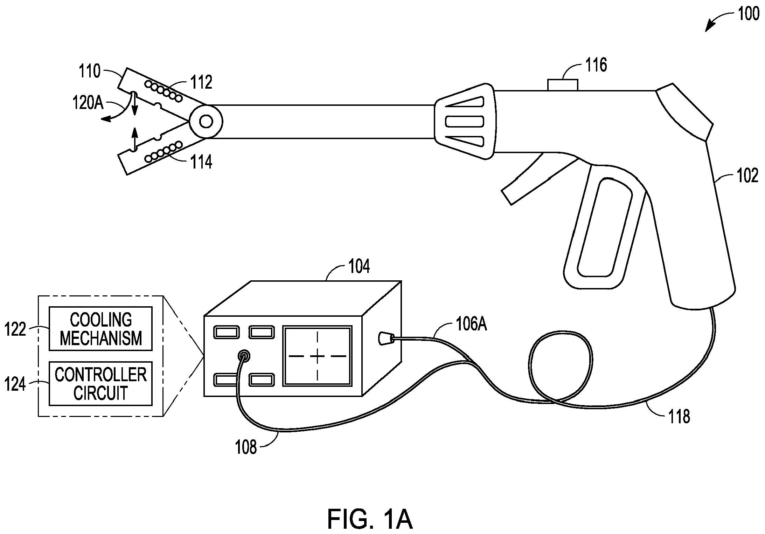

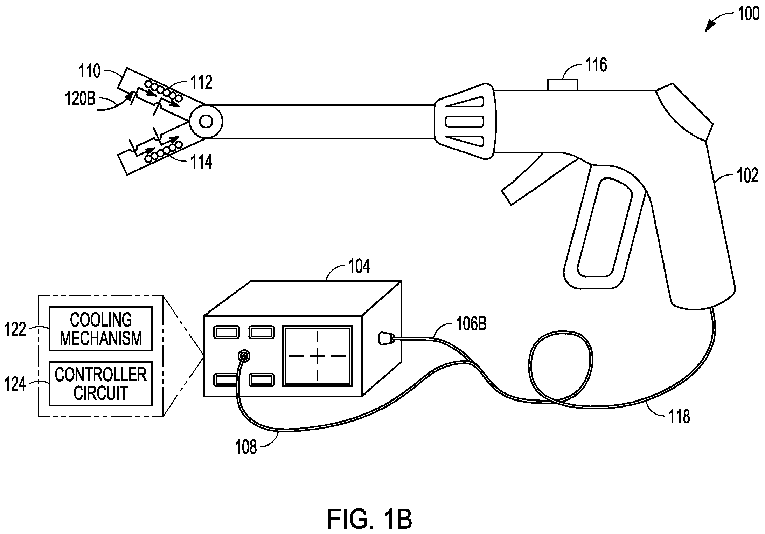

FIGS. 1A and 1B illustrate an example of a system 100 such as may include a handheld electrosurgical device 102. The electrosurgical device 102 can include an electrosurgical end effector 110, such as can be connected to an electrosurgical generator 104 via a signal coupling means such as a line 118. The line 118 may include one or more electrically conducting wires 108 such as to communicate an electrosurgical or other electrical signal from the generator 104 to the end effector 110. The system may include a cooling apparatus 122, which may be included within or a part of the electrosurgical generator 104, or which may be separately provided and controlled by or in concert with the electrosurgical generator 104.

The cooling apparatus 122 may include a refrigeration system, such as a cryonic or cryogenic device, a thermoelectric cooler, a heat pump, or the like, such as to chill at least a portion of the device directly or indirectly, such as via irrigation fluid such as saline or via a heat pipe, or both. The irrigation fluid may be passed from the cooling apparatus 122 through irrigation line 106A toward the end effector 110 so as to provide cooling irrigation at or near the end effector 110. The heat pipe may be configured to conduct heat between the desired portion of the device to be cooled and the refrigeration system, or between the refrigeration system and an intermediate location or substance, such as the irrigation fluid. In an example, both the irrigation line 106A and a heat pipe can be used to transfer heat such as to cool the end effector 110. In an example such as shown in FIG. 1B, the cooling apparatus 122 may include a suction pump, such as which may be connected to a suction line 106B, such as to exhaust heated gas or liquid fluid, or other substance from a location at or near the end effector 110 via the suction line 106B, such as to help lower the temperature at or near the end effector 110.

The end effector 110 may include an opening such as 120A or 120B to provide liquid or other fluid irrigation at or near the end effector 110 or to allow heated gas or liquid fluid or other substance to be removed from a location at or around the end effector 110, such as via suction.

The end effector 110 may include a thermal sensor 112 such as a thermocouple, thermistor, a fiber optic sensor, or other temperature sensor. The end effector 110 may also include an electrical heater 114. The heater 114 may include a resistive heater, such as can include a resistive material such as a metal such as nickel or chromium, a composite heating element such as nichrome, among others, through which a current can be passed, such as can be provide from the electrosurgical generator 104, such as to actuate and provide heat via the heater 114.

Activating the irrigation line 106A, the suction line 106B, or the heater 114 may be controlled via an activation actuator or trigger 116, which may include a switch, button, or other actuator or trigger. This can allow the surgeon to control actively heating or cooling the end effector 110 as needed during a surgical procedure. In an example, activation of the heater 114, the irrigation line 106A, or the suction line 106B, may be controlled automatically, such as without requiring user intervention, such as using a controller circuit 124, which may be included as a part of the electrosurgical generator 104, that can monitor the temperature at the end effector 110 using the thermal sensor 112.

For example, the temperature at the end effector 110 may be controlled or stabilized by using the thermal sensor 112 to monitor the temperature at the end effector 110. A reading of the measured temperature at the end effector 110 can be relayed to the controller circuit 124. The controller circuit 124 may use a comparator or other device to compare the monitored temperature at the end effector 110 with a pre-set temperature threshold value or range. The controller circuit 124 can activate or otherwise control operation of the heater 114 or the cooling apparatus 122 such as in response to the comparison of the monitored temperature. Such control by the controller circuit 124 can include activating or controlling the irrigation line 106A or the suction line 106B, such as to adjust or change the temperature at the end effector 110. For example, the controller circuit may initiate, activate, or otherwise control a valve, or a similar actuator, which can cause irrigation fluid to flow or to stop flowing through the irrigation line 106A based on the monitored temperature. In another example, the controller circuit may control the flow of irrigation fluid or the amount of suction such that the fluid flow or amount of suction is increased, decreased, or otherwise controlled, to an amount as needed, based on the measured temperature.

When the monitored temperature is above a first temperature limit, the cooling apparatus 122 can be activated or initiated or increased. Likewise, when the monitored temperature is below a second temperature limit the heater 114 can be activated or initiated or increased. The controller circuit 124 may activate the heater 114 or the cooling apparatus 122 when energy is not being applied to the end effector 110, such as when the jaws of a surgical forceps are in an open position. For example, when the jaws of a surgical forceps are closed, or when energy is being applied to the end effector 110, the heater 114 or the cooling apparatus 122 may be locked out, or otherwise prevented from being engaged, activated, turned on, or the like, while the end effector 110 is actively being used, such as to manipulate tissue of a patient. Then, when the jaws of the surgical forceps are open, or energy to the end effector 110 is stopped, disengaged, turned off, or the like, the heater 114 or the cooling apparatus 122 may be engaged such as manually by a user, or by the controller circuit 124 in response to the measured temperature at the end effector 110.

The controller circuit 124 can allow the temperature at the end effector 110 to be maintained within a temperature range, band, or the like (e.g., such as between 40 and 50 degrees Celsius). This can provide a way to keep the temperature at the end effector 110 stable, such as within a specified temperature range that can help limit the chance of thermal effects on the patient's tissue, e.g., such as either from too much active heating or from too much active cooling. This, in turn, may be useful in a variety of medical procedures. For example, the electrosurgical system 100 may be used in medical procedures which require properly detecting a size of a blood vessel, such as procedures which require sealing a blood vessel. In such procedures determining the size of a vessel may be done by measuring an initial impedance of the vessel, which can be affected by the temperature at the end effector 110. For example, the impedance measurement may be different when the temperature of the end effector 110 is at body temperature than it is when the temperature is above body temperature. Thus, a surgeon may desire to keep the temperature of the end effector 110 within a pre-selected range.

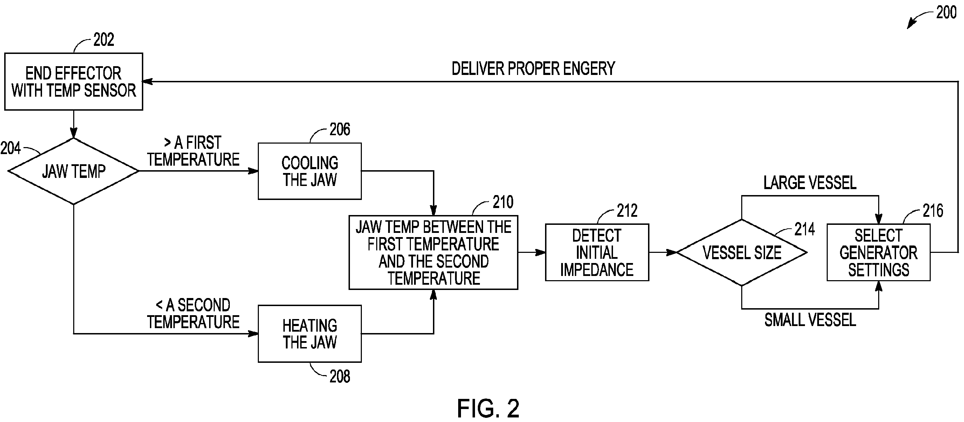

FIG. 2 illustrates a flowchart showing a technique 200 for determining the size of a blood vessel by stabilizing the jaw temperature of a handheld electrosurgical apparatus and detecting an initial impedance. 202 includes an end effector such as 110 with a temperature sensor such as 112 to measure a temperature at the end effector, such as at the jaws of a surgical forceps. 204 includes measuring the jaw temperature of the end effector. When the jaw temperature measured at 204 is above a first temperature, the jaw is cooled at 206, such as by the controller circuit 124 as described above for FIGS. 1A and 1B, activating a cooling apparatus such as 122. When the jaw temperature measured at 204 is below a second temperature, the jaw is heated at 208 such as by the controller circuit 124 activating the heater 114 such as described above. An indication of temperature status can be provided to the user in response to the temperature measurement, such as to inform the user of whether the measured temperature is above or below one or more threshold values, or whether the measured temperature is within or outside of a specified temperature range. Different indications of temperature status can be used to inform the user of different measured temperature conditions. For example, when the jaw temperature measured at 204 is above the first threshold temperature, a first type of indicator can be provided to the user (such as lighting an LED or other visual display using a first color (e.g., red) or visual indicator, providing a second type of haptic feedback, or both). The first and second indicators may be different or distinct from each other such as different colored LEDs, so as to be distinguishable from the other. When the jaw temperature measured at 204 is between the first and second threshold temperatures, a third type of indicator can be provided to the user (such as lighting an LED or other visual display using a third color (e.g., green) or visual indicator, providing an absence of haptic feedback, or both).

In an example, an indicator may be audible, such as emitting a sound through a speaker coupled, attached, or otherwise connected to, or included on or within a portion of the handheld electrosurgical apparatus. In an example, the indication can be made by displaying a message on a graphical user interface (GUI), such as on a liquid crystal display (LCD) or another similar display attached, connected, or coupled to the apparatus. In an example, multiple indicators of different types as described above, or other similar indicators (e.g., audible, visual, haptic, or the like) may be used in one or more combinations to provide the user the different indications of temperature status.

Returning to FIG. 2, at 210 the temperature of the jaw is determined to be between the first temperature and the second temperature, for example, between 40 C and 50 C. When the jaw temperature is measured to be between the first temperature and the second temperature, an initial impedance value is detected at 212. Based on the initial impedance value at 212, the size of the vessel is determined at 214.

Once the size of the vessel is determined at 214, at 216 the proper generator settings are selected to send an electrical signal from an electrosurgical generator such as 102, to the end effector, returning to 202 in order to repeat the process as needed.

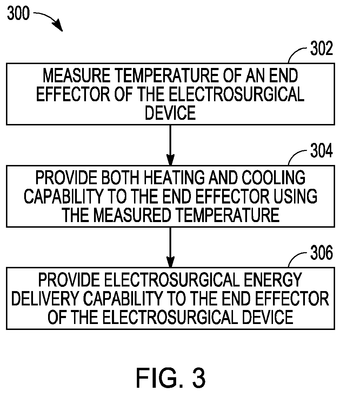

FIG. 3 illustrates an example comprising a technique, such as a method 300 for using an electrosurgical apparatus. At 302 the temperature at the end effector of an electrosurgical device, such as 110 in FIGS. 1A and 1B is measured. The temperature may be measured using a temperature sensor such as 112 included on the end effector 110. Returning to FIG. 3, 304 may include providing both heating and cooling to an end effector using the measured temperature from 302. This may include cooling using a cryogenic cooler, an air or other gas flow cooler, a fluid flow cooler, a thermoelectric cooler, an active suction cooler, a fluid flushing cooler, a closed-circuit fluid flow cooler, a treatment site irrigation conduit, a heat pipe, or the like.

Heat may be provided to the end effector by one or more of a resistive heater, a heat pipe, a heat pump, an electrosurgical electrode, or other similar heating or heat transport element. In an example, heating or cooling of the end effector may be controlled manually by a user of the electrosurgical apparatus, such as through an activation member (e.g., a switch or button located on the apparatus). In another example, heat or cooling provided to the end effector at 302 may be controlled automatically, such as by a controller circuit within an electrosurgical generator which activates a heating element, or a cooling apparatus based on a measured temperature at the end effector as described above for FIGS. 1A and 1B.

At 306 electrosurgical energy can be provided to the end effector. This may be done in response to the jaw temperature being within a temperature range or band, between a first temperature and a second temperature. One of the first temperature and the second temperature may represent a lower limit, and the other of the first temperature and the second temperature may represent an upper limit. When the temperature at the end effector is between the lower limit and the upper limit, in response to heating or cooling the end effector as desired, electrosurgical energy can be provided to the end effector to perform a desired surgical procedure on a tissue of a patient.

The above detailed description includes references to the accompanying drawings, which form a part of the detailed description. The drawings show, by way of illustration, specific embodiments that may be practiced. These embodiments are also referred to herein as "examples." Such examples may include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

All publications, patents, and patent documents referred to in this document are incorporated by reference herein in their entirety, as though individually incorporated by reference. In the event of inconsistent usages between this document and those documents so incorporated by reference, the usage in the incorporated reference(s) should be considered supplementary to that of this document; for irreconcilable inconsistencies, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are openended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Further examples of the present disclosure can be found in the following paragraphs:
Example 1: An electrosurgical apparatus comprising:
   an electrosurgical end effector;
   a temperature sensor to measure a temperature of the end effector;
   a cooler, to cool the end effector based on the measured temperature; and
   a heater, to heat the end effector based on the measured temperature.
Example 2. The apparatus of example 1, wherein each of the temperature sensor, the cooler, and the heater are configured to be coupled to a controller circuit that is configured to use the heater to heat the end effector when the measured temperature is below a first temperature limit and to use the cooler to cool the end effector when the measured temperature is above a second temperature limit.
Example 3. The apparatus of example 2, further including the controller circuit.
Example 4. The apparatus of example 2, wherein the heater is activated automatically when the measured temperature is below the first temperature limit.
Example 5. The apparatus of example 2, wherein the cooler is activated automatically when the measured temperature is above the second temperature limit.
Example 6. The apparatus of example 1, wherein the end-effector includes at least one of a jaw or forceps including or coupled to the temperature sensor, the cooler, and the heater.
Example 7. The apparatus of example 1, including an irrigation conduit separate from the cooler.
Example 8. The apparatus of example 1, wherein the cooler includes at least one of a cryogenic cooler, an air or other gas flow cooler, a fluid flow cooler, a thermoelectric cooler, an active suction cooler, a fluid flushing cooler, a closed-circuit fluid flow cooler, a treatment site irrigation conduit, or a heat pipe.
Example 9. The apparatus of example 1, wherein the heater includes a resistive heater.
Example 10. The apparatus of example 1, further comprising a first range indicator to notify a user of whether the measured temperature is within a range.
Example 11. The apparatus of example 10, wherein the first range indicator comprises a first optical indicator.
Example 12. The apparatus of example 10, wherein the first range indicator comprises a second optical indicator.
Example 13. The apparatus of example 10, wherein the first range indicator comprises a first audible indicator.
Example 14. The apparatus of example 10, wherein the first range indicator comprises a second audible indicator.
Example 15. The apparatus of example 10, wherein the first range indicator comprises a first haptic indicator.
Example 16. The apparatus of example 10, wherein the first range indicator comprises a second haptic indicator.
Example 17. The apparatus of example 1, further comprising a second range indicator to notify a user of whether the measured temperature is outside a range.
Example 18. The apparatus of example 17, wherein whether the measured temperature is outside a range includes:
   when the measured temperature is below a first temperature limit; and
   when the measured temperature is above a second temperature limit.
Example 19. The apparatus of example 17, wherein the second range indicator includes one or more of: an optical indicator, a haptic indicator, or an audible indicator.
Example 20. A method of using an electrosurgical device, the method comprising: measuring a temperature of an end effector of the electrosurgical device;
   providing both heating and cooling capability to the end effector, controlled using the measured temperature; and
   providing electrosurgical energy delivery capability to the end effector of the electrosurgical device.
Example 21. The method of example 20, comprising actively heating and cooling the end effector based on the measured temperature.
Example 22. The method of example 20, wherein the end effector includes at least one of a forceps, a spatula, a blade, an electrode, or a needle.
Example 23. The method of example 20, comprising controlling the heating to heat the end effector when a temperature of the end effector is less than a first temperature value, and controlling the cooling to cool the end effector when the temperature of the end effector is greater than a second temperature value.
Example 24. The method of example 20, comprising alerting a user to notify the user of at least one of the measured temperature or whether the measured temperature is within a range.
Example 25. The method of example 24, wherein alerting the user comprises providing haptic feedback to the user.
Example 26. The method of example 20, comprising controlling at least one of the heating or cooling based on a positional state of the end effector.
Example 27. The method of example 26, comprising performing at least one of heating or cooling when jaws of the end effector are in an open position.
Example 28. An electrosurgical apparatus comprising:
   means for measuring a temperature of an end effector of the electrosurgical apparatus;
   means for both heating and cooling the end effector, in response to a signal that is based on the measured temperature; and
   means for providing electrosurgical energy delivery capability to the end effector.
Example 29. The apparatus of example 28, further comprising control circuitry configured for actively heating and cooling the end effector based on the measured temperature.

The above description is intended to be illustrative, and not restrictive. For example, the above-described examples (or one or more aspects thereof) may be used in combination with each other. Other embodiments may be used, such as by one of ordinary skill in the art upon reviewing the above description. The Abstract is to allow the reader to quickly ascertain the nature of the technical disclosure and is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description, with each claim standing on its own as a separate embodiment. The scope of the embodiments should be determined with reference to the appended claims, along with the full scope of equivalents to which such claims are entitled.

## Claims

1. An electrosurgical apparatus comprising:
an electrosurgical end effector;
a temperature sensor to measure a temperature of the end effector;
a cooler, to cool the end effector based on the measured temperature; and
a heater, to heat the end effector based on the measured temperature.

2. The apparatus of claim 1, wherein each of the temperature sensor, the cooler, and the heater are coupleable to a controller circuit to control the heater to heat the end effector when the measured temperature is below a first temperature limit and to control the cooler to cool the end effector when the measured temperature is above a second temperature limit.

3. The apparatus of claim 2, further including the controller circuit.

4. The apparatus of claim 2 or 3, wherein the heater is activated automatically when the measured temperature is below the first temperature limit.

5. The apparatus of any of claims 2 to 4, wherein the cooler is activated automatically when the measured temperature is above the second temperature limit.

6. The apparatus of any of claims 1 to 5, wherein the end-effector includes at least one of a jaw or forceps including or coupled to the temperature sensor, the cooler, and the heater.

7. The apparatus of any of claims 1 to 6, including an irrigation conduit separate from the cooler.

8. The apparatus of any of claims 1 to 7, wherein the cooler includes at least one of a cryogenic cooler, an air or other gas flow cooler, a fluid flow cooler, a thermoelectric cooler, an active suction cooler, a fluid flushing cooler, a closed-circuit fluid flow cooler, a treatment site irrigation conduit, or a heat pipe.

9. The apparatus of any of claims 1 to 8, wherein the heater includes a resistive heater.

10. The apparatus of any of claims 1 to 9, further comprising a first range indicator to notify a user of whether the measured temperature is within a range.

11. The apparatus of claim 10, wherein the first range indicator comprises one or more optical indicators, one or more audible indicators, and/or one or more haptic indicators.

12. The apparatus of any of claims 1 to 11, further comprising a second range indicator to notify a user of whether the measured temperature is outside a range.

13. The apparatus of claim 12, wherein the second range indicator includes one or more of: an optical indicator, a haptic indicator, or an audible indicator.

14. An electrosurgical apparatus comprising:
means for measuring a temperature of an end effector of the electrosurgical apparatus;
means for both heating and cooling the end effector, in response to a signal that is based on the measured temperature; and
means for providing electrosurgical energy delivery capability to the end effector.

15. The apparatus of claim 14, further comprising control circuitry configured for actively heating and cooling the end effector based on the measured temperature.
